# EUROPÄISCHE PATENTSCHRIFT

(11) **EP 2 987 468 B1**
(45) Veröffentlichungstag und Bekanntmachung des Hinweises auf die Patenterteilung: **07.08.2019**
(21) Anmeldenummer: 15187936.8
(22) Anmeldetag: 10.06.2011
(51) Int. Cl.: A61F 2/46

(54) **VAKUUMZEMENTIERSYSTEM**
VACUUM CEMENTATION SYSTEM
SYSTEME DE CIMENTATION SOUS VIDE

(30) Priorität: 07.07.2010 DE 102010026497
(43) Veröffentlichungstag der Anmeldung: 24.02.2016
(62) Teilanmeldung aus: 11752460.3
(73) Patentinhaber: Heraeus Medical GmbH, 61273 Wehrheim (DE)
(72) Erfinder: Vogt, Sebastian, 99092 Erfurt (DE); Büchner, Hubert, 90491 Nürnberg (DE); Schnieber, Tim, 60439 Frankfurt (DE)
(74) Vertreter: Heraeus IP

(56) Entgegenhaltungen:
- EP-A1- 1 741 413
- JP-A- H0 857 284
- US-A1- 2003 155 381
- US-B1- 7 073 936

## Beschreibung

Die Erfindung betrifft ein Vakuumzementiersystem mit einem Behälteröffnungssystem zum Öffnen von Behältern.

Die Erfindung stellt Vakuumzementiersysteme zum Öffnen von Monomerampullen für Polymethylmethacrylat-Knochenzemente (PMMA-Knochenzemente), die mit Monomerflüssigkeit vorgefüllt sind, bereit, die als Full-Prepack-Vakuumzementiersysteme dem medizinischen Anwender zur Verfügung gestellt werden.

PMMA-Knochenzemente sind seit Jahrzehnten bekannt und gehen auf die grundlegenden Arbeiten von Sir Charnley zurück (Charnley, J.: Anchorage of the femoral head prosthesis of the shaft of the femur. J. Bone Joint Surg. 42 (1960) 28-30.). Der Grundaufbau der PMMA-Knochenzemente ist seit dem prinzipiell gleich geblieben. PMMA-Knochenzemente bestehen aus einer flüssigen Monomerkomponente und einer Pulverkomponente. Die Monomerkomponente enthält im Allgemeinen das Monomer Methylmethacrylat und einen darin gelösten Aktivator (N,N-Dimethyl-p-toluidin). Die Pulverkomponente besteht aus einem oder mehreren Polymeren, die auf Basis von Methylmethacrylat und Comonomeren, wie Styren, Methylacrylat oder ähnlichen Monomeren durch Polymerisation, vorzugsweise Suspensionspolymerisation, hergestellt sind, einem Röntgenopaker und dem Initiator Dibenzoylperoxid. Beim Vermischen der Pulverkomponente mit der Monomerkomponente entsteht durch Quellung der Polymere der Pulverkomponente im Methylmethacrylat ein plastisch verformbarer Teig. Bei dem Vermischen der Pulverkomponente mit der Monomerkomponente reagiert der Aktivator N,N-Dimethyl-p-Toluidin mit Dibenzoylperoxid unter Bildung von Radikalen. Die gebildeten Radikale initiieren die radikalische Polymerisation des Methylmethacrylates. Mit fortschreitender Polymerisation des Methylmethacrylates erhöht sich die Viskosität des Zementteigs bis dieser erstarrt.

Polymethylmethacrylat-Knochenzemente können in geeigneten Mischbechern mit Hilfe von Spateln durch Vermischen des Zementpulvers mit der Monomerflüssigkeit vermischt werden. Nachteilig ist an dieser Vorgehensweise, dass Lufteinschlüsse im gebildeten Zementteig vorhanden sein können, die später eine Destabilisierung des Knochenzementes verursachen können. Aus diesem Grund wird das Vermischen von Knochenzementpulver mit der Monomerflüssigkeit in Vakuummischsystemen bevorzugt, weil durch Mischen im Vakuum Lufteinschlüsse aus dem Zementteig weitgehend entfernt werden und damit eine optimale Zementqualität erreicht wird (Breusch SJ et al.: Der Stand der Zementiertechnik in Deutschland. Z Orthop. 1999, 137: 101-07). Im Vakuum gemischte Knochenzemente haben eine deutlich verringerte Porosität und zeigen daher verbesserte mechanische Eigenschaften. Es wurden eine Vielzahl von Vakuumzementiersystemen offen gelegt, von denen exemplarisch folgende genannt sind:
US 5,624,184, US 4,671,263, US 4,973,168, US 5,100,241, WO 99/67015 A1,
EP 1 020 167 A2, US 5,586,821, EP 1 016 452 A2, DE 36 40 279 A1, WO 94/26403 A1,
EP 0 692 229 A1, EP 1 005 901 A2, US 5,344,232.

Vakuumzementiersysteme werden beim Mischen des PMMA-Knochenzements unter Vakuum gesetzt, um Lufteinschlüsse aus dem Zementteig zu entfernen. Es soll dadurch ein möglichst homogener, weitgehend blasenfreier Zementteig gebildet werden.

Eine Weiterentwicklung stellen Zementiersysteme dar, in denen sowohl das Zementpulver als auch die Monomerflüssigkeit bereits in separaten Kompartimenten der Mischsysteme verpackt sind und erst unmittelbar vor der Zementapplikation im Zementiersystem miteinander vermischt werden (US 5,997,544, EP 0 692 229 A1, US 6,709,149 B1). Problematisch ist bei diesen Systemen die Überführung der Monomerflüssigkeit in das Zementpulver und die vollständige Vermischung dieser beiden Komponenten, damit ein homogener Zementteig erhalten wird, der insbesondere keine Nester an nicht von der Monomerflüssigkeit benetzten Zementpulver enthalten darf. Bei dem gegenwärtig in Europa auf dem Markt befindlichen Full-Prepack-Mischsystem Optipac™ (Biomet Switzerland) wird durch einfache Röhren, die seitlich im unteren Teil der Kartusche angebracht sind, welche die Kartuschenwand durchstoßen, die Monomerflüssigkeit ungefähr in der Mitte des Zementpulvers durch Einwirkung von Vakuum aus Aluminiumverbundbeuteln in das Zementpulver eingesaugt. Aluminiumverbundbeutel werden zum Öffnen manuell gegen die Röhren bewegt, wobei die Röhren die Wandung der Beutel durchstechen.

Aluminiumverbundbeutel sind für die Verpackung und Lagerung von Monomerflüssigkeit erst seit wenigen Jahren bekannt. Sehr gute Erfahrungen hinsichtlich der Lagerfähigkeit von Monomerflüssigkeit liegen mit Glasampullen vor. Glasampullen werden seit Jahrzehnten für konventionelle Polymethylmethacrylat-Knochenzemente mit gutem Erfolg eingesetzt. Glasampullen haben neben der perfekten Dichtigkeit auch den Vorteil, dass sie in großen Stückzahlen zu niedrigen Preisen gefertigt werden können. Daher ist es sinnvoll, Glasampullen zur Verpackung und Lagerung von Monomerflüssigkeit in Prepack-Vakuumzementiersystemen einzusetzen.

In der DE 195 32 015 A1 ist eine Vorrichtung zum Mischen und Ausbringen von Mehrkomponentenprodukten beschrieben. Dabei wird eine Vorrichtung zum Öffnen von Ampullen vorgeschlagen, die darauf beruht, dass an der Außenseite der Zementkartusche eine Lagerbuchse ausgebildet ist, um die sich drehbar ein Ampullenhalter bewegen kann. Der Kopf der Ampulle befindet sich im Inneren der Lagerbuchse. Bei der Drehung des Ampullenhalters um die Lagerbuchse wird der Ampullenkopf vom Ampullenkörper abgeschert. Dann kann die Flüssigkeit aus der Ampulle in die Kartusche durch eine Öffnung in der Kartuschenwand überführt werden.

In der WO 97/18031 A1 wird eine Vorrichtung vorgeschlagen, bei der eine Ampulle am Boden durchstoßen wird und die Monomerflüssigkeit danach durch den hohlen Mischstab in die Zementkartusche fließen kann.

Ein System zum Öffnen von Ampullen bei Zementiersystemen wird in der EP 1 031 333 A1 offen gelegt. Bei diesem System wird durch eine Bewegung des Mischstabs gegen eine keilförmige Vorrichtung im Kartuschenkopf der Ampullenkopf schräg gegen die Ampullenachse bewegt, wobei der Ampullenkopf vom Ampullenkörper abgeschert wird. Problematisch sind hierbei der relativ komplexe Aufbau der Öffnungsvorrichtung und die Gefahr des Verklemmens der keilförmigen Vorrichtung.

In der WO 2010/012114 A1 ist eine Vorrichtung zum Öffnen von Ampullen beschrieben. Dabei handelt es sich analog zur DE 195 32 015 A1 um einen Drehmechanismus zum Abscheren des Ampullenkopfs. Der einzige Unterschied zur DE 195 32 015 A1 besteht darin, dass eine Drehbuchse gegen den Ampullenhalter bewegt wird und nicht wie bei der DE 195 32 015 A1 der Ampullenhalter gegen die Drehbuchse.

Es kann also zusammenfassend festgestellt werden, dass alle bisher bekannten Prepack-Vakuumzementiersysteme manuell zu öffnende Monomerflüssigkeitsbehälter enthalten. Vorteilhafter wäre es, wenn beim Anlegen des Vakuums an das Zementiersystem eine zwangsweise automatische Öffnung der Monomerflüssigkeitsbehälter erfolgen würde. Dadurch wäre die Handhabung der Vakuumzementiersysteme für den medizinischen Anwender deutlich vereinfacht und sicherer.

US-A-2003/0155381 offenbart ein Vakuumzementiersystem, bei dem eine Ampulle durch das angelegte Vakuum auf einen Keil zubewegt wird und dadurch aufgebrochen wird.

Der Erfindung liegt die Aufgabe zu Grunde, ein Full-Prepack-Vakuumzementiersystem zu entwickeln, bei dem Monomerflüssigkeitsbehälter ohne manuelle Betätigung automatisch geöffnet werden und damit der Monomertransfer aus einem Monomerbehälter zu einem Zementbehälter automatisch ausgelöst werden kann.

Diese Aufgabe wird durch ein Vakuumzementiersystem mit mindestens einer Zementpulverkartusche und einem Behälteröffnungssystem gelöst, wobei das Behälteröffnungssystem wenigstens einen Behälter für zumindest eine Flüssigkeit und/oder zumindest ein Pulver, eine Öffnungsvorrichtung und eine Auslassöffnung zum Anlegen eines Unterdrucks umfasst, wobei bei Anlegen eines Unterdrucks die Öffnungsvorrichtung relativ zu zumindest einem Teil des Behälters beweglich ist, und das Vakuumzementiersystem ein Mittel zur Verbindung der Zementpulverkartusche mit dem Behälter umfasst, wobei das für die Herstellung eines blasenarmen Zementteigs benötigte Vakuum zusätzlich zum automatischen Öffnen der Behälter genutzt wird,
dadurch gekennzeichnet, dass
der Behälter eine flexible Wand umfasst und die Öffnungsvorrichtung ein Dorn oder eine Klinge ist, wobei der Behälter beweglich im Behälteröffnungssystem angeordnet ist und/oder die Wand verformbar ist und der Behälter oder ein Teil des Behälters durch Druckdifferenz auf eine statische Öffnungsvorrichtung gedrückt wird und der Differenzdruck direkt und unmittelbar auf den beweglichen Behälter wirkt, so dass sich der Behälter gegen das Behälteröffnungssystem drückt, wobei es sich bei dem Behälter um einen Folienbeutel handelt und eine Wand des Folienbeutels gasdicht an einem die Auslassöffnung bildenden Rohr anliegt und im Inneren des Rohrs ein Dorn mit eine Schneide angeordnet ist, derart, dass bei Anlegen eines Unterdrucks der Folienbeutel in das Innere des Rohrs gesaugt wird und von der Schneide des Dorns aufgeschlitzt wird.

Dadurch wird zumindest ein Teil einer Wand des Behälters relativ zu einem an dem Behälteröffnungssystem befestigten Dorn, der als Öffnungsvorrichtung wirkt, bewegt wird und dabei die Wand des Behälters aufgeschnitten oder eingestochen und dadurch geöffnet wird.

Auch kann vorgesehen sein, dass der oder die Behälter eine Flüssigkeit, insbesondere eine Monomerflüssigkeit, und/oder ein Pulver, insbesondere ein Zementpulver, beinhalten, wobei der Inhalt nach dem Öffnen aus dem Behälter unter Einwirkung des Unterdrucks, insbesondere eines Vakuums, austritt und anschließend mit einem Pulver, insbesondere einem Zementpulver, oder einer Flüssigkeit, insbesondere einer Monomerflüssigkeit, unter Einwirkung des Unterdrucks, insbesondere eines Vakuums, durchmischt wird.

Dabei kann vorgesehen sein, dass das Gemisch, vorzugsweise mit Hilfe eines Überdrucks, aus einem Applikator herausgedrückt wird oder herausläuft.

Ein Behälteröffnungssystem, das zum Durchführen eines solchen Verfahrens geeignet ist, umfasst wenigstens einen Behälter für zumindest eine Flüssigkeit und/oder zumindest ein Pulver, eine Öffnungsvorrichtung und eine Auslassöffnung zum Anlegen eines Unterdrucks.

Erfindungsgemäß ist vorgesehen, dass der Behälter eine vorzugsweise flexible Wand umfasst und die Öffnungsvorrichtung ein Dorn oder eine Klinge ist, wobei der Behälter beweglich im Behälteröffnungssystem angeordnet ist und/oder die Wand verformbar ist.

Es kann auch vorgesehen sein, dass der Behälter eine Flüssigkeit umfasst, die eine Monomerkomponente, vorzugsweise ein polymerisierbares Monomer, wie zum Beispiel Methylmethacrylat, und einen darin gelösten Aktivator, enthält.

Bei dem Behälter handelt es sich um einen Monomerflüssigkeitsbehälter.

Dabei kann vorgesehen sein, dass die Zementpulverkartusche mindestens ein Mischorgan, insbesondere einen Förderkolben umfasst.

Es kann auch vorgesehen sein, dass ein statischer Mischer an einer Applikationsöffnung des Vakuumzementiersystems angeordnet ist.

Es kann ferner vorgesehen sein, dass die Zementpulverkartusche ein Pulver umfassend ein oder mehrere Polymere, insbesondere auf der Basis von Methylmethacrylat und Comonomeren, beinhaltet.

Unter einem mittelbaren Öffnen eines Behälters durch die Druckdifferenz ist zu verstehen, dass ein bewegliches Mittel, wie zum Beispiel ein Kolben, durch den äußeren Druck relativ zu dem Behälter und auch relativ zu dem gesamten Behälteröffnungssystem bewegt wird und diesen dadurch mechanisch öffnet, also beispielsweise durch Aufbrechen, Aufschneiden oder Einstechen eines Teils des Behälters beziehungsweise einer Behälterwand. Dazu wird das Mittel zum Öffnen des Behälters beweglich im Behälteröffnungssystem gelagert. Der Differenzdruck wirkt also auf das Mittel zur Öffnung, was in einer Bewegung des Mittels resultiert.

Ein unmittelbares Öffnen ist dann gegeben, wenn ein Behälter oder ein Teil eines Behälters durch die Druckdifferenz auf eine statische Öffnungsvorrichtung gedrückt wird. Der Differenzdruck wirkt hierbei also direkt und unmittelbar auf den beweglichen Behälter, so dass sich der Behälter gegen das Behälteröffnungssystem bewegt. Der Behälter ist dazu beweglich im Behälteröffnungssystem gelagert. Der Behälter wird dann auf eine Öffnungsvorrichtung, die mit dem Behälteröffnungssystem verbunden ist, geschoben beziehungsweise gedrückt und durch Aufbrechen, Aufschneiden oder Einstechen eines Teils des Behälters beziehungsweise einer Behälterwand mechanisch geöffnet.

Eine mittelbare Öffnung ist auch dann gegeben, wenn sich sowohl der Behälter als auch die Öffnungsvorrichtung bewegen, also beide beweglich im Behälteröffnungssystem gelagert sind.

Die Erfindung basiert auf der überraschenden Erkenntnis, dass ein Vakuum bei Vakuumzementiersystemen neben der Entfernung von Lufteinschlüssen aus dem Zementteig auch zum Öffnen von Behältern, die beispielsweise ein Monomer beinhalten, nutzbar gemacht werden kann. Somit wird das bei Vakuumzementiersystemen ohnehin für die Herstellung eines blasenarmen Zementteigs benötigte Vakuum zusätzlich auch zum automatischen Öffnen von Behältern, beispielsweise Monomerflüssigkeitsbehältern, genutzt. Durch die Erzeugung eines Unterdrucks im Zementiersystem wird die entstehende Druckdifferenz zur Umgebung zum Antrieb eines automatischen, mechanischen Öffnungssystems verwendet. Durch diese Krafteinwirkung wird der Behälter geöffnet und dessen Inhalt, zum Beispiel ein Monomer, das zur Bildung von Knochenzement erforderlich ist, freigesetzt.

Unter dem Begriff Behälter im Sinne der Erfindung werden vorzugsweise Kunststoffverbundbeutel, einschließlich Aluminiumverbundbeutel, verstanden. Die Anmeldung offenbart unter dem Begriff Behälter auch Glasampullen, Kunststoffampullen und Aluminiumampullen.

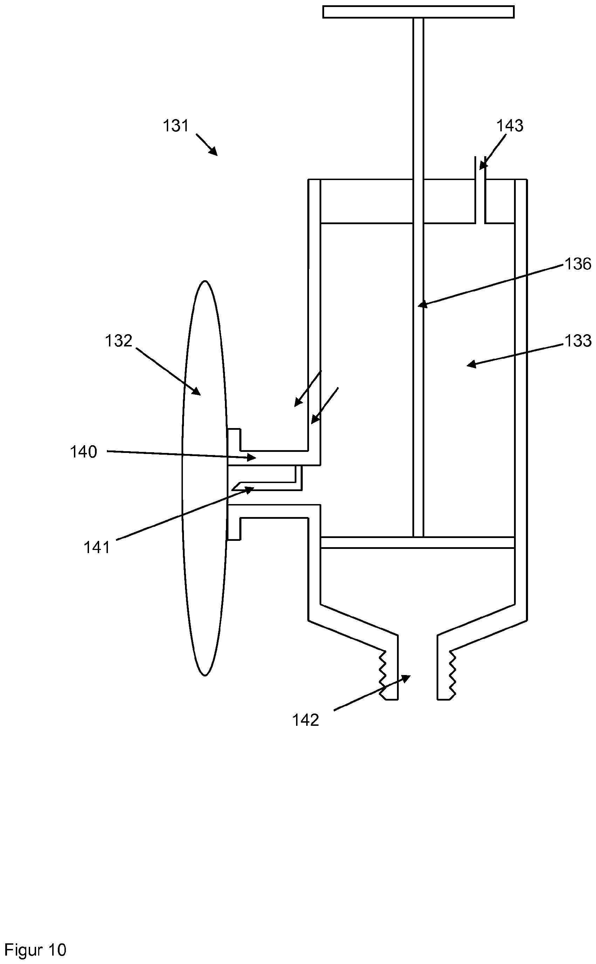

Im Folgenden werden Ausführungsbeispiele anhand von zehn schematisch dargestellten Zeichnungen erläutert, wobei Figur 10 eine erfindungsgemäße Ausführung zeigt. Dabei zeigen:
- Figur 1:: eine schematische Querschnittansicht in Längsrichtung eines Behälteröffnungssystems mit geschlossenem Behälter;
- Figur 2:: Eine schematische Aufsicht auf einen Kolben mit gabelförmiger Führung als Öffnungsvorrichtung für ein Vakuumzementiersystem;
- Figur 3:: Eine schematische Vorderansicht des Kolbens nach Figur 2;
- Figur 4:: Eine schematische perspektivische Ansicht des Kolbens nach Figur 2;
- Figur 5:: Eine schematische Querschnittansicht in Längsrichtung eines alternativen Vakuumzementiersystems mit geschlossenem Behälter;
- Figur 6:: Eine schematische Querschnittansicht in Längsrichtung eines drittenVakuumzementiersystems mit geschlossenem Behälter;
- Figur 7:: Eine schematische Querschnittansicht in Längsrichtung eines viertenVakuumzementiersystems mit geschlossenem Behälter;
- Figur 8:: Eine schematische Querschnittansicht in Längsrichtung eines fünftenVakuumzementiersystems mit geschlossenem Behälter;
- Figur 9:: Eine schematische Querschnittansicht eines Vakuumzementiersystems; und
- Figur 10:: Eine schematische Querschnittansicht in Längsrichtung eines sechsten,erfindungsgemäßen Vakuumzementiersystems mit geschlossenem Behälter.

Beispiele für offenbarte und erfindungsgemäße Vakuumzementiersysteme sind in den folgenden Figurenbeschreibungen erläutert.

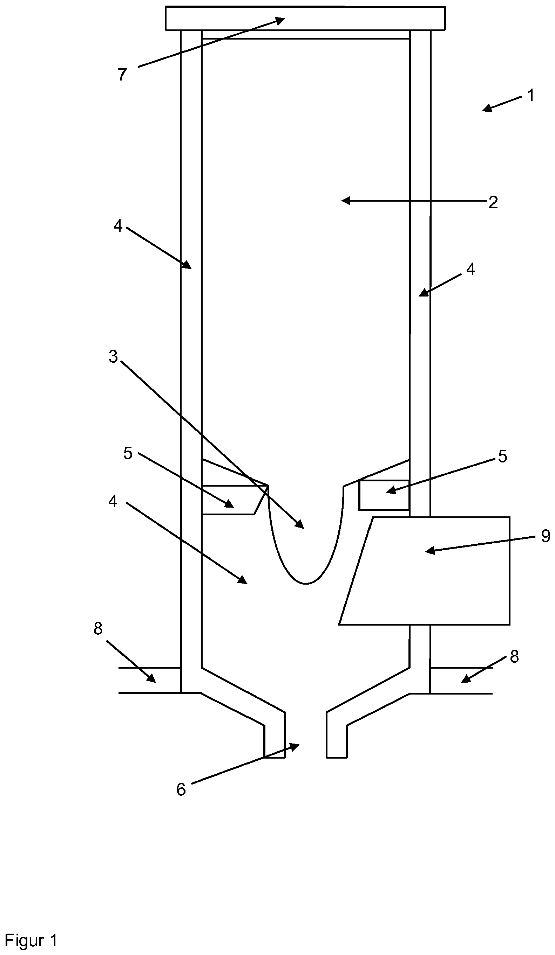

Figur 1 zeigt eine schematisch dargestellte Querschnittansicht eines Behälteröffnungssystems (1), in dem ein Behälter (2) in Form einer Ampulle angeordnet ist. Der Behälter (2) umfasst einen Behälterkopf (3) und gemäß Figur 1 einen darüber befindlichen Behälterkörper. Zwischen dem Behälterkopf (3) und dem Behälterkörper befindet sich eine Brechzone.

Der Behälter ist in einer Kammer (4) des Behälteröffnungssystems (1) angeordnet und durch zwei Stutzen (5) positioniert. Unterhalb des Behälterkopfs (3) ist eine Auslassöffnung (6) in der Kammer (4) vorgesehen. Die Oberseite der Kammer (4) ist durch einen Deckel (7) verschlossen. Der Deckel (7) ist durch ein Gewinde oder einen anderen Verschlussmechanismus mit der Kammer (4) verbunden. Das Behälteröffnungssystem (1) kann in einer anderen Vorrichtung, die hier durch die offenen Wände (8) dargestellt ist, eingebaut oder integriert sein.

Im Bereich des Behälterkopfs (3) ist eine zylindrische Öffnung in den Wänden der Kammer (4) vorgesehen, in der ein zylindrischer Kolben (9) entlang seiner Symmetrieachse beweglich angeordnet ist. Die Seite des Kolben (9), die in das Innere der Kammer (4) reicht, ist abgeschrägt. Die Schräge soll dafür sorgen, dass der Behälterkopf (3) leicht vom Behälterkörper abbricht, wenn der Kolben (9) ins Innere der Kammer (4) hineingeschoben wird. Dem gleichen Zweck dient die Abschrägung des dem Kolben (9) gegenüberliegenden Stutzens (5). Durch beide Maßnahmen soll die Kraft, die durch den Kolben (9) wirkt, auf die Brechzone konzentriert und verstärkt werden.

Die Wände der Kammer (4) und der Deckel (7) sind druckstabil, erhalten also ihre Form auch dann, wenn im Inneren der Kammer (4) ein Vakuum erzeugt wird. Der Kolben (9) steckt in gasdichter Presspassung in der Öffnung der Wände der Kammer (4).

Das Verfahren kann beispielsweise wie folgt durchgeführt werden: Zunächst wird an der Auslassöffnung (6) ein Unterdruck angelegt. Dadurch bildet sich auch im Inneren der Kammer (4) ein Unterdruck aus. Die Druckdifferenz zwischen dem Inneren der Kammer (4) und der äußeren Umgebung der Kammer (4) führt dazu, dass auf der nach außen weisenden Fläche des Kolbens (9) ein größerer Druck und damit eine größere Kraft wirkt, als auf der nach innen weisenden Fläche des Kolbens (9). Der Kolben (9) erfährt daher eine resultierende Kraft, die ihn ins Innere der Kammer (4) treibt. Sobald die Haftreibung des in Presspassung sitzenden Kolbens (9) durch die resultierende Kraft überwunden ist, bewegt sich der Kolben (9) ins Innere der Kammer (4). Schließlich wird der Kolben (9) auf den Behälterkopf (3) treffen und diesen abscheren. Hierdurch wird der Behälter (2) geöffnet. Der Inhalt des Behälters (2) fließt aus diesem heraus und durch die Auslassöffnung (6) hindurch aus dem Behälteröffnungssystem (1) ab.

Da bei neuartigen Vakuumzementiersystemen ohnehin ein Vakuum erzeugt wird, um beispielsweise eine Monomerflüssigkeit mit einem Knochenzement unter Vakuum zu mischen, kann dieses Vakuum auch gleichzeitig dazu genutzt werden, den steril abgeschlossenen Behälter (2), in dem sich die Monomerflüssigkeit und/oder das Knochenzementpulver befindet, zu öffnen. Geeignete und geläufige Behälter (2) für die Monomerflüssigkeit beziehungsweise auch den Knochenzement sind beispielsweise Glasampullen.

Der Haftreibungswiderstand des Kolbens (9) in der Öffnung der Kammer (4) und/oder die Stabilität der Brechzone des Behälters (2) beziehungsweise der Querschnitt des Kolbens (9) können dabei so eingestellt werden, dass sich der Behälter (2) beziehungsweise die Glasampulle erst dann öffnet, wenn der Unterdruck in der Kammer (4) dazu ausreicht, ein ausreichend blasenfreies Gemisch aus Monomerflüssigkeit und Knochenzement zu erzeugen.

Die Öffnungsvorrichtung stellt in diesem Ausführungsbeispiel der beweglich gelagerte Kolben (9) dar.

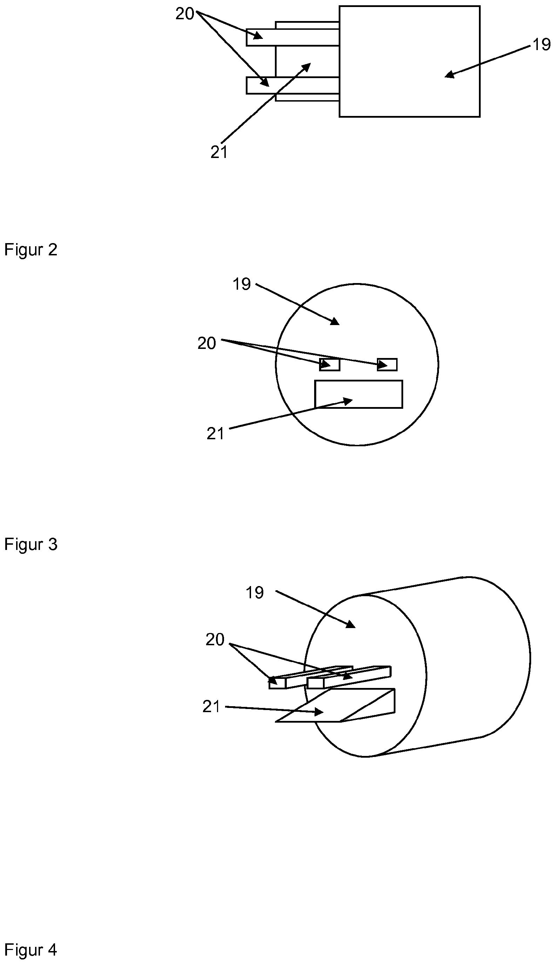

Die Figuren 2, 3 und 4 zeigen in verschiedenen Ansichten schematische Darstellungen eines alternativ gestalteten Kolbens (19) für alternative Behälteröffnungssysteme (nicht gezeigt). Der Kolben (19), der als Öffnungsvorrichtung für Behälter fungieren soll, umfasst eine gabelförmige Führung bestehend aus zwei Stegen (20) und umfasst des Weiteren eine abgeschrägte vorstehende Platte (21). Die gabelförmige Führung (20) soll die Bewegung des Kolbens (19) relativ zu einem Behälterkopf definieren. Ein hierfür geeigneter Behälter hat eine Verjüngung zwischen dem Behälterkopf und dem Behälterkörper, in den die gabelförmige Führung (20) greifen kann.

Mit der Platte (21) kann entweder ein Behälterkopf abgebrochen beziehungsweise abgeschert werden oder die Kante der Platte (21) ist so scharf, dass sie in der Lage ist, die Wände eines Behälters zu durchschneiden. Die gabelförmige Führung (20) und die Platte (21) dienen dazu, den Öffnungsvorgang reproduzierbarer zu machen und genauer durchführen zu können. Der Zwischenraum zwischen den beiden Stegen (20) kann sich zum Kolben (19) hin verjüngen, um dadurch ein Abtrennen eines Behälterkopfs bei der Bewegung des Kolbens (19) zu ermöglichen. Die Platte (21) ist dann nicht mehr notwendig.

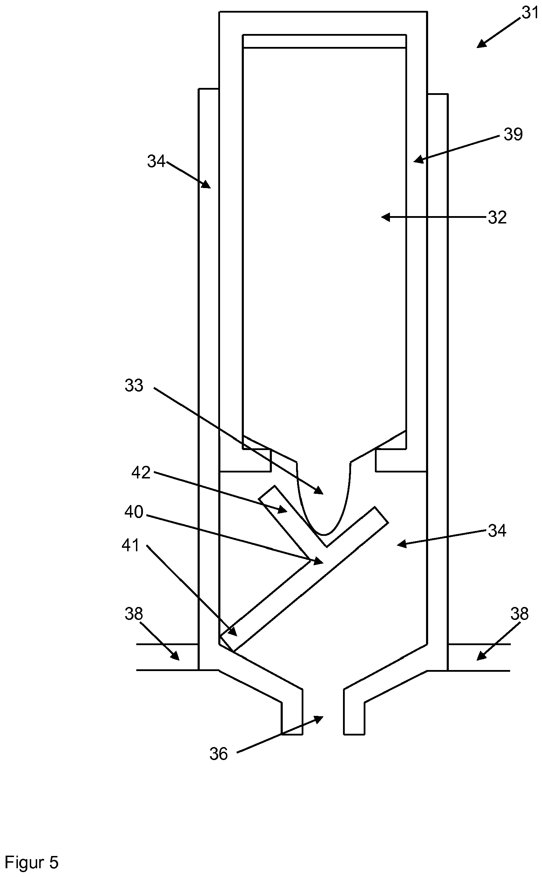

Figur 5 zeigt eine weitere schematische Querschnittansicht eines zweiten alternativen Behälteröffnungssystems (31). Das Behälteröffnungssystem (31) umfasst einen Behälter (32) mit einem Behälterkopf (33). In dem Behälter (32) kann beispielsweise eine Flüssigkeit enthalten sein. Der Behälter (32) befindet sich im Inneren eines Kolbens (39), der wiederum beweglich in einer Kammer (34) steckt. Am unteren Ende der Kammer (34) befindet sich eine Auslassöffnung (36) unter dem Behälterkopf (33). Das gesamte Behälteröffnungssystem (31) ist in eine Vorrichtung, die durch die offenen Wände (38) dargestellt ist, wie beispielsweise ein Vakuumzementiersystem, integriert.

Im Inneren der Kammer (34) befindet sich ein drehbar gelagerter Hebel (40) als Öffnungsvorrichtung für den Behälter (33), der mit einem ersten Hebelarm (41) an den Wänden der Kammer (34) gelagert ist, wobei ein zweiter Hebelarm (42) in Form eines senkrecht vom ersten Hebelarm (41) abzweigenden Stegs lose an den Behälterkopf (33) anlehnt.

Bei einem offenbarten Verfahren wird in der Kammer (34) ein Vakuum erzeugt, indem die Luft über die Auslassöffnung (36) abgepumpt wird. Aufgrund des Differenzdrucks zwischen dem Inneren der Kammer (34) und der Umgebung des Behälteröffnungssystems (31) wird der bewegliche Kolben (39) mit dem Behälter (32) in die Kammer (34) hineingezogen. Dies führt gleichzeitig zu einer Drehung des Hebels (40), wodurch eine Kraft durch den zweiten Hebelarm (42) auf den Behälterkopf (33) ausgeübt wird. Sobald diese Kraft ausreicht, wird der Behälterkopf (33) zerbrochen oder vom Behälter (32) abgebrochen.

Die in dem Behälter (32) gelagerte Flüssigkeit kann aus dem Behälter (33) austreten und durch die Auslassöffnung (36) aus dem Behälteröffnungssystem (31) auslaufen. Anschließend kann die Flüssigkeit mit Hilfe des an der Auslassöffnung (36) anliegenden Unterdrucks einem Zementpulver (nicht gezeigt) zugeführt werden, und im Vakuumzementiersystem (durch die offenen Wände (38) angedeutet) mit dem Zementpulver gemischt werden.

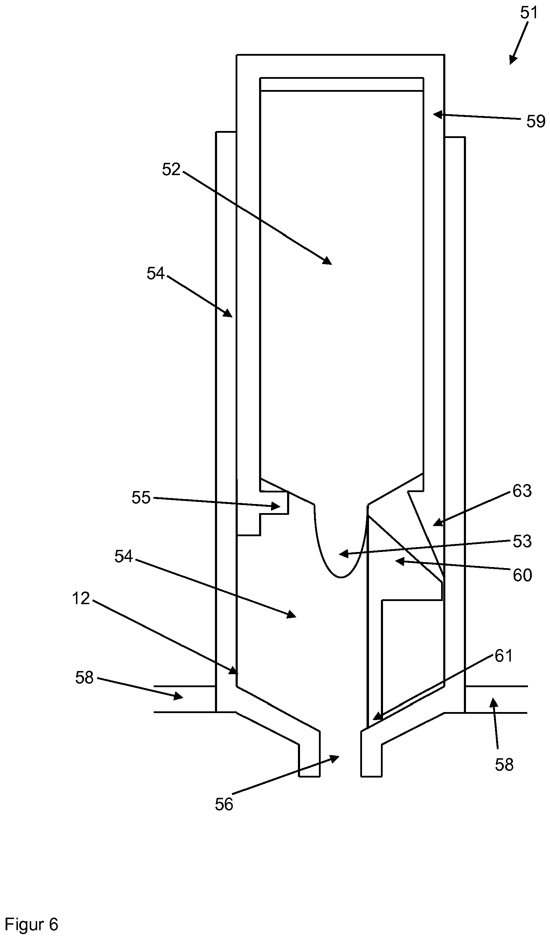

Figur 6 zeigt eine schematische Querschnittansicht eines dritten Behälteröffnungssystems (51). Wie in dem zweiten Ausführungsbeispiel befindet sich ein Behälter (52) im Inneren eines Kolbens (59), der in einer gasdichten Öffnung einer Kammer (54) beweglich angeordnet ist. Gegenüber eines Behälterkopfs (53) befindet sich in der Kammer (54) eine Auslassöffnung (56), über die ein Unterdruck an der Kammer (54) angelegt werden kann.

Der Kolben (59) umfasst auf der einen Seite einen Stutzen (55) zur Halterung des Behälters (52) und auf der anderen Seite einen keilförmigen Steg (63) mit einer Schrägung auf der dem Behälter (52) zugewandten Seite. In der Kammer (54) befindet sich ein in Längsrichtung zum Behälter (52) ausgebildeter Hebel (60). Der Hebel (60) ist im Bereich der Auslassöffnung (56) über einen ersten Hebelarm (61) fest mit den Wänden der Kammer (54) verbunden und weist auf der dem Behälter (52) abgewandten Seite eine Schrägung auf. Der erste Hebelarm (61) ist aus einem elastisch leicht verformbaren Material gefertigt.

Wird die Kammer (54) mit Vakuum beaufschlagt, so wird der Kolben (59) mit dem darin enthaltenen Behälter (52) ins Innere der Kammer (54) gezogen. Die Schräge des keilförmigen Stegs (63) schiebt sich zwischen die Wände der Kammer (54) und die Schräge des Hebels (60). Dadurch wird der Hebel (60) seitlich gegen den Behälterkopf (53) gedrückt, wodurch dieser vom Behälter (52) abbricht. Die Spitze des Hebels (60) kann zusätzlich in die Sollbruchstelle des sich in die Kammer (54) hinein bewegenden Behälters (52) stechen beziehungsweise schneiden.

Es kann also vorgesehen sein, dass der Kolben (59) einen parallel zur Ampullenachse ausgeformten keilförmigen Steg (63) besitzt, wobei eine Schrägung auf der zur Ampullenseite zugewandten Seite vorgesehen ist, und sich in der Kammer (54) ein in Längsrichtung zur Ampullenachse ausgebildeter Hebel (60) befindet, dessen oberes Hebelende sich frei beweglich neben dem Ampullenkopf (53) befindet, wobei der Hebel (60) auf der der Ampullenachse abgewandten Seite eine Schrägung besitzt und der Hebel (60) mit dem unteren Hebelende (61) beweglich mit der Kammer (54) verbunden ist.

Bei Beaufschlagung mit Vakuum bewegt sich der Steg (63) zusammen mit den Kolben (59) in Richtung Ampullenkopf (53), wobei die Schrägung des Stegs (63) auf die Schrägung des Hebels (60) drückt. Der Hebel (60) ist an seinem unteren Hebelende (61) fixiert und führt mit seinem beweglichen oberen Hebelende zwangsweise eine Drehbewegung gegen den Ampullenkopf (53) aus. Dadurch bricht der Ampullenkopf (53) vom Ampullenkörper ab. Bei dieser mittelbaren Öffnung des Behälters wirkt der Keil (63) zusammen mit dem Hebel (60) als Öffnungsvorrichtung.

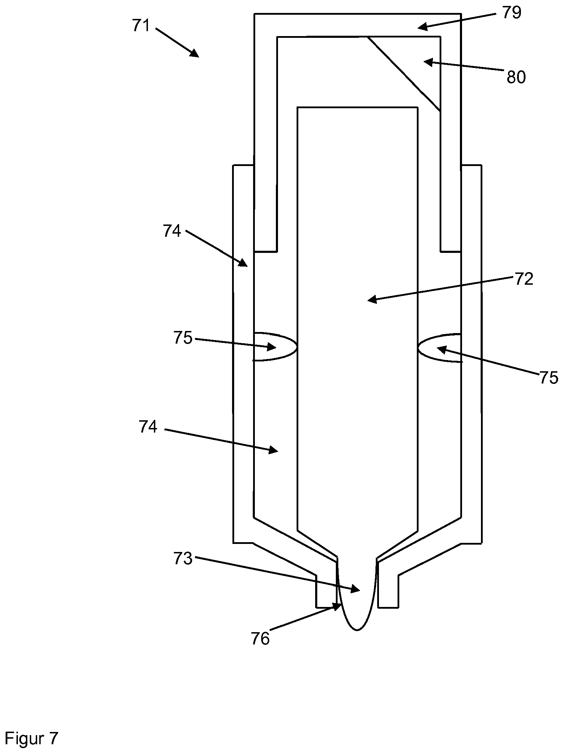

Figur 7 zeigt eine weitere schematische Querschnittansicht eines Behälteröffnungssystems (71) mit einem in einer Kammer (74) beweglich angeordneten Kolben (79) in einer Öffnung an der Oberseite der Kammer (74). Die Außenwände des Kolbens (79) schließen gasdicht mit der Öffnung der Kammer (74) ab. Ein Behälter (72) ist innerhalb der Kammer (74) durch Stutzen (75) an den Wänden der Kammer (74) und durch eine Auslassöffnung (76) im Boden der Kammer (74) positioniert. In der Auslassöffnung (76) steckt ein Behälterkopf (73) des Behälters (72), der über eine Sollbruchstelle mit dem Behälterkörper verbunden ist.

Die Stutzen (75) bestehen aus einem festen Material und stützen den Behälter (72) im Wesentlichen nur punktweise. Eine Stützung entlang Kontaktlinien oder Flächen ist jedoch auch möglich. Die Berührungspunkte der Stutzen (75) definieren einen Drehpunkt, um den der Behälter (72) gekippt werden kann.

Der bewegliche Kolben (79) ist ein Hohlkörper mit einseitig geschlossenem Ende. Der Innenquerschnitt des Kolbens (79) ist größer als der Außenquerschnitt des Behälters (72). Im Inneren des Kolbens (79) befindet sich ein Steg (80) in Form einer Schräge, die am geschlossenen Ende und an einer Zylinderwand des Kolbens (79) angeordnet ist. Der Behälter (72) ragt bereichsweise in den Kolbens (79) hinein.

Die Form des Behälterkopfs (73) und die Form der Auslassöffnung (76) sind derart aneinander angepasst, dass sie nicht formschlüssig miteinander schließen, das heißt, dass die Form des Behälterkopfs (73) nicht genau der Form der Auslassöffnung (76) entspricht. Hierdurch soll erreicht werden, dass die Auslassöffnung (76) nicht vollständig durch den Behälterkopf (73) geschlossen ist.

So ist es möglich, dass durch die Auslassöffnung (76) die Atmosphäre aus dem Inneren der Kammer (74) evakuiert werden kann, und so ein Unterdruck in der Kammer (74) erzeugt wird. Der in der Umgebung des Behältersystems (71) herrschende Normaldruck schiebt dann den Kolben (79) in Richtung der Auslassöffnung (76).

Der Steg (80) am geschlossenen Ende des Kolbens (79) wird dann gegen eine obere Ecke des Behälters (72) geschoben und bei weiterem Hineindrücken des Kolbens (79) wird der Behälter (72) gekippt. Da der Behälterkopf (73) der Kippbewegung des Behälters (72) nicht folgen kann, zerbricht er oder er bricht ab und der Inhalt des Behälters (72) ergießt sich durch die Auslassöffnung (76).

Als Öffnungsvorrichtung wirkt hier also der Steg (80) mit dem Kolben (79), sowie die Halterung des Behälterkopfs (76) zusammen mit den Stutzen (75). Es ist in diesem Fall also gar nicht ohne weiteres auszumachen, welche Teile die Öffnungsvorrichtung bilden. Letztendlich ist meist ein Zusammenspiel verschiedener Komponenten nötig, damit eine Öffnung des Behälters erreicht wird. Diese Komponenten bilden dann die Öffnungsvorrichtung.

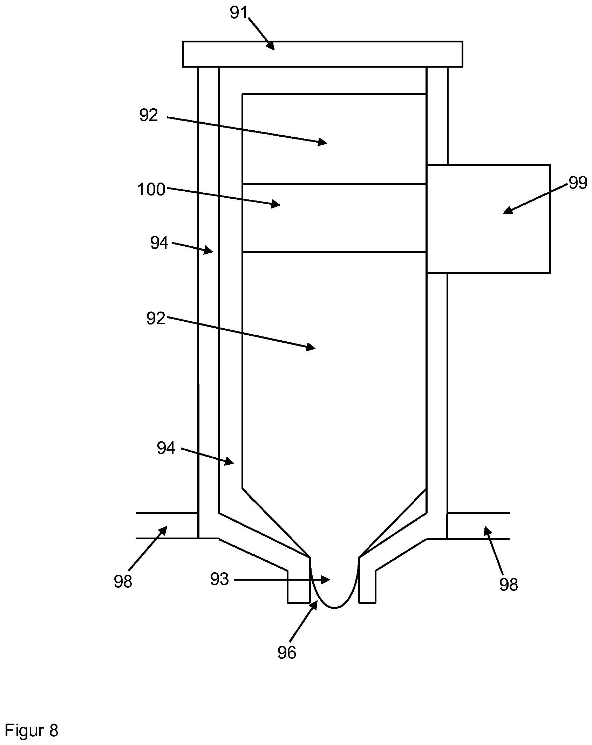

Figur 8 zeigt eine weitere Ausführungsform eines Behälteröffnungssystems (91). Bei diesem Ausführungsbeispiel erfolgt das Öffnen eines Behälters (92) wieder durch Abbrechen oder Zerbrechen eines Behälterkopfs (93), der in einer Auslassöffnung (96) einer Kammer (94) gehalten wird. Die Kammer (94) ist durch einen Deckel (97) geschlossen. Der Deckel (97) verschließt die Kammer (94) gasdicht und vakuumdicht.

Das gesamte Behälteröffnungssystem (91) ist in ein Vakuumzementiersystem (angedeutet durch die offenen Wände (98)) integriert.

In einer Öffnung der Wände der Kammer (94) befindet sich ein in der Öffnung beweglicher Kolben (99), der die Öffnung gasdicht abschließt. Der Kolben (99) ist an den Behälter (92) mit einer Halterung (100) in Form einer Manschette befestigt.

Die Auslassöffnung (96) ist in diesem Fall nicht zentrisch in der Kammer (94) angeordnet. Dadurch liegt der Behälter (92) auf der Seite mit den Kolben (99) dicht an den Außenwänden der Kammer (94) an, während auf der gegenüberliegenden Seite noch Platz zu den Wänden der Kammer (94) ist.

Dadurch wird erreicht, dass bei einem Eindringen des Kolbens (99) in die Kammer (94) der Behälter (92) um eine Achse in der Verbindung des Behälterkopfs (91) zum Behälterkörper gedreht wird. Da der Behälterkopf (91) diese Bewegung nicht mitgehen kann, wird er abgebrochen oder zerbrochen. Dadurch wird der Behälter (92) geöffnet. Der Inhalt des Behälters (92) kann durch die Auslassöffnung (96) austreten und steht einem Vakuumzementiersystem, das an das Behälteröffnungssystem (91) angeschlossen ist, zum Mischen mit einer anderen Komponente zur Verfügung.

Der Kolben (99) wird wieder aufgrund eines Unterdrucks in der Kammer (94) bewegt. Zur Verwendbarkeit der Auslassöffnung (96) ist es wichtig, dass der Behälterkopf (93) und der Behälter (92) die Auslassöffnung (96) nicht verschließen. Nur dann ist sichergestellt, dass die Auslassöffnung (96) sowohl zum Evakuieren der Kammer (94) als auch zum Abführen des Inhalts des Behälters (92) verwendet werden kann.

Alternativ kann auch eine zweite freie Öffnung neben der Auslassöffnung (96) in der Kammer (94) vorgesehen sein, über die die Kammer (94) evakuiert werden kann und über die auch der Inhalt des Behälters (92) aus der Kammer (94) austreten kann. Statt in der Auslassöffnung (96) gehalten zu werden, kann auch einfach eine separate Befestigung für den Behälterkopf (93) in der Kammer (94) vorgesehen sein, die keine Verbindung zum Äußeren der Kammer hat.

Die Öffnungsvorrichtung wird hier durch den Kolben (99), die Halterung (100) und die Halterung des Behälterkopfs (93), das heißt die Auslassöffnung (96) gebildet und erzwingt eine mittelbare Öffnung des Behälters (92).

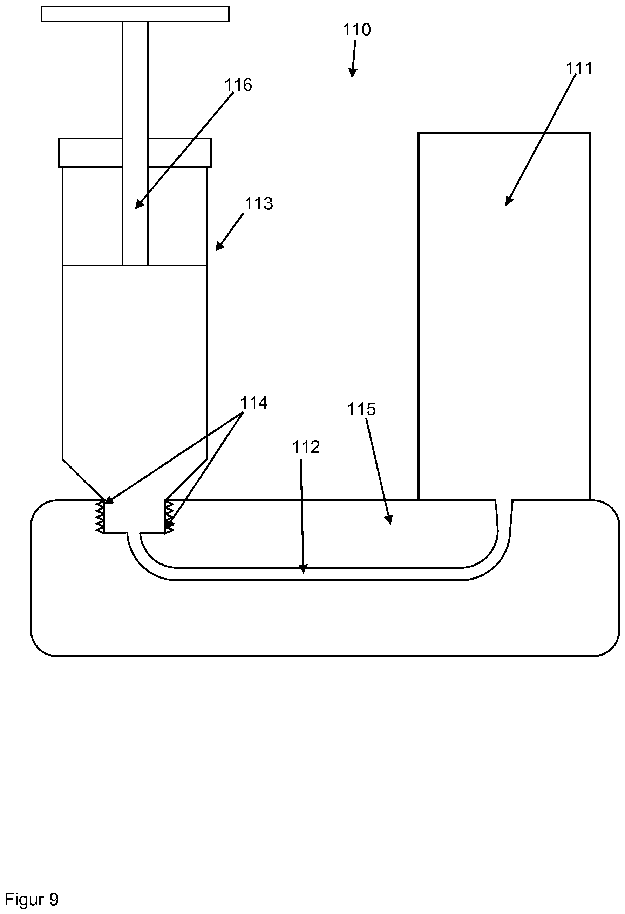

Figur 9 zeigt ein Vakuumzementiersystem (110) umfassend ein Behälteröffnungssystem (111) umfassend einen mit einer Flüssigkeit gefüllte Glasampulle als Behälter (nicht gezeigt), die über ein Verbindungsmittel (112), wie beispielsweise eine Leitung, mit einer Zementpulverkartusche (113) verbunden ist. Die Zementpulverkartusche (113) ist über Befestigungsmittel (114), wie beispielsweise einem Gewinde, mit einem Fußteil (115) des Vakuumzementiersystems (110) verbunden. Die Zementpulverkartusche (113) umfasst einen Förderkolben (116), mit dem der Inhalt der Kartusche (113) aus dem Vakuumzementiersystem (110) ausgetrieben beziehungsweise der fertig gemischte Zement appliziert werden kann.

Mit Hilfe eines Vakuums wird zunächst die Glasampulle im Behälteröffnungssystem (111), wie in den anderen Figuren gezeigt, geöffnet. Die Flüssigkeit aus dem Behälter wird durch das Vakuum über das Verbindungsmittel (112) in die Zementpulverkartusche (113) gesaugt und vermischt sich dort mit dem Zement. Mit Hilfe des Förderkolbens (116) kann das Gemisch appliziert werden. Ein statischer Mischer (nicht gezeigt) kann in der Applikationsöffnung (nicht gezeigt) des Vakuumzementiersystems (110) vorgesehen sein, um eine stärkere Durchmischung der Flüssigkeit mit dem Zement zu erreichen.

Besonders vorteilhaft sind das Verfahren und das Vakuumzementiersystem (111) für PMMA-Knochenzemente geeignet, wobei als Flüssigkeit eine Monomerkomponente (die beispielsweise Methylmethacrylat und einen Aktivator enthält) und als Pulverkomponente ein aus einem oder mehreren Polymeren bestehendes Knochenzementpulver (beispielsweise auf der Basis von Methylmethacrylat und Comonomeren) verwendet wird beziehungsweise in dem Behälter und der Zementpulverkartusche (113) enthalten sind.

Figur 10 zeigt ein erfindungsgemäßes Vakuumzementiersystem (130) umfassend ein, im Vergleich zu den anderen Ausführungsbeispielen etwas anderen Behälteröffnungssystem (131). Das Behälteröffnungssystem (131) umfasst einen Behälter (132) in Form eines Folienbeutels, der eine Flüssigkeit beinhaltet, und ein Rohr (140), wobei im Inneren des Rohrs (140) ein Dorn (141) mit einer Schneide angeordnet ist. Eine Wand des Folienbeutels (132) liegt gasdicht an dem Rohr (140) an. Der Dorn (141) ist an einer der Rohrwände befestigt. Das Rohr (140) entspricht in begrenzter Weise den Auslassöffnungen (6, 36, 56, 76, 96) und dem Verbindungsmittel (112) in anderen Ausführungsbeispielen.

Auf der dem Behälter (132) gegenüberliegenden Seite des Rohrs (140) ist eine Zementpulverkartusche (133) mit dem Rohr (140) verbunden. Im Inneren der Zementpulverkartusche (133) befindet sich ein Förderkolben (136), mit dem der Inhalt der Zementpulverkartusche (133) aus einer Applikationsöffnung (142) ausgetrieben werden kann. An einem Stutzen der Applikationsöffnung (142) befindet sich ein Befestigungsmittel (134) in Form eines Gewindes, an dem eine Applikatorspitze mit einem statischen Mischer (nicht gezeigt) anschließbar ist. Am oberen Ende der Zementpulverkartusche (133) ist ein Vakuumanschluss (143) angeordnet, an den eine Vakuumpumpe beziehungsweise eine Unterdruckquelle anschließbar ist.

Bei der erfindungsgemäßen Ausführungsform des Verfahrens wird zunächst ein Unterdruck an dem Vakuumanschluss (143) angelegt. Der Folienbeutel (132) wird in das Innere des Rohrs (140) gezogen und von der Schneide des Dorns (141) aufgeschlitzt. Der Inhalt des Behälters (132) wird in das Rohr (140) gesaugt. Gleichzeitig wird der Förderkolben (136) nach oben gezogen. Schließlich kann die Flüssigkeit aus dem Behälter (132) durch das Rohr (140) in die Zementpulverkartusche (133) gelangen. Dort vermischt sie sich mit einem in der Zementpulverkartusche (133) enthaltenen Zementpulver.

Durch manuelles drücken auf den Förderkolben (136) oder durch Anlegen eines Überdrucks an den Vakuumanschluss (143) kann der Förderkolben anschließend wieder in Richtung der Applikationsöffnung (142) geschoben werden. Dabei wird das Gemisch Zementpulver/Flüssigkeit aus der Applikationsöffnung (142) ausgetrieben.

Bei der erfindungsgemäßen Ausführungsform nach Figur 10 wird eine unmittelbare Öffnung des Behälters (132) erreicht. Der Behälter (132) selbst bewegt sich mit der im Rohrquerschnitt (140) anliegenden flexiblen Wand auf die fest mit dem Behälteröffnungssystem verbundene Öffnungsvorrichtung, nämlich den Dorn (141). Ebenso wäre es vorstellbar, dass der Behälter (132) starre Behälterwände hat und in dem Rohr (140) selbst angeordnet ist. Damit er auf den Dorn (141) gesaugt werden kann, muss er, wie die Kolben (9, 39, 59, 79, 99) in den anderen Ausführungsbeispielen, gasdicht mit den Wänden des Rohrs (140) abschließen.

Es ist auch erfindungsgemäß, wenn die Wände des Behälters (132) im Bereich des Rohrs (140) aufgrund des Innendrucks des Behälters (132) selbst reißen. Im Rohr (140) kann ein Ventil vorgesehen sein, dass ein Rücklaufen des Zements in das Rohr (140) und in den Behälter (132) verhindert.

Für alle Ausführungsbeispiele gilt, dass unterhalb der Auslassöffnung (6, 36, 56, 76, 96) beziehungsweise in dem Rohr (140) ein Sieb und/oder ein Filter (nicht gezeigt) angeordnet sein kann, mit dem Splitter beziehungsweise Reste des Behälters (2, 32, 52, 72, 92, 132), die beim Aufschneiden, Abschneiden oder Zerbrechen des Behälters (2, 32, 52, 72, 92, 132) beziehungsweise beim Abbrechen des Behälterkopfs (3, 33, 53, 73, 93) entstehen können, zurückgehalten werden.

Der Behälter (2, 32, 52, 72, 92, 132) ist vorzugweise eine Ampulle, insbesondere eine Glasampulle.

Ein Verfahren zur Öffnung von Ampullen in Vakuumzementiersystemen kann auch dadurch gekennzeichnet sein, dass durch Einwirkung eines Vakuums mit einem absoluten Druck von größer gleich 70 mbar auf mindestens einen Kolben, der Kolben relativ zu einem oder mehreren Monomerflüssigkeitsbehälter(n) verschoben wird, und dass dadurch der oder die Monomerflüssigkeitsbehälter geöffnet werden, wobei die Öffnung durch Einstechen und/oder Aufschneiden und/oder Aufbrechen erfolgt.

Gezeigt sind auch Vorrichtungen umfassend mindestens eine Zementpulverkartusche (113, 133) mit mindestens einem Mischorgan (116, 136), eine Ampulle (1, 31, 51, 71, 91, 11, 131) als Monomerflüssigkeitsbehälter, ein Verbindungsmittel (112) zur Verbindung der Zementpulverkartusche (113, 133), ein Ampullenbehälter, gebildet in mindestens einer Kammer (4, 34, 54, 74, 94) und mindestens ein Kolben (9, 19, 39, 59, 79, 99), wobei
a) mindestens ein Kolben (9, 19, 39, 59, 79, 99) derart angeordnet ist, dass mindestens eine Kammer (4, 34, 54, 74, 94) gegen die äußere, unter Normaldruck stehende Umgebung abgegrenzt ist,
b) der Kolben (9, 19, 39, 59, 79, 99) gasdicht mit der Kammer (4, 34, 54, 74, 94) verbunden ist,
c) der Querschnitt der Kammer (4, 34, 54, 74, 94) so ist, dass der Kolben (9, 19, 39, 59, 79, 99) entlang der Kolbenachse durch die Kammer (4, 34, 54, 74, 94) verschoben werden kann,
d) der Kolben (9, 19, 39, 59, 79, 99) entlang der Kolbenachse verschiebbar angeordnet ist,
e) der Kolben (9, 19, 39, 59, 79, 99) mit seiner der Kammer (4, 34, 54, 74, 94) zugewandten Seite mindestens an einer Stelle mit der in dieser Kammer (4, 34, 54, 74, 94) angeordneten Ampulle (2, 32, 52, 72, 92, 132) kraftschlüssig und/oder formschlüssig verbindbar ist,
f) die Kammer (4, 34, 54, 74, 94) mindestens eine Auslassöffnung (6, 36, 56, 76, 96) für Fluide enthält und/oder
g) der Kolben (9, 19, 39, 59, 79, 99) durch Veränderung des Verhältnisses zwischen dem Druck in der Kammer (4, 34, 54, 74, 94) und dem Druck der Umgebung entlang seiner Kolbenachse bewegbar ist und dabei eine Kraft auf die Ampulle (2, 32, 52, 72, 92, 132) ausübt.

Dabei kann ferner vorgesehen sein, dass der Kolben (9, 19, 39, 59, 79, 99) senkrecht zur Ampullenachse angeordnet ist und an der Kolbenseite, die in Richtung des Inneren der Kammer (4, 34, 54, 74, 94) gewandt ist, senkrecht oder schräg zur Wandfläche der Kammer (4, 34, 54, 74, 94) ausgebildet ist, wobei die Kolbenseite eine glatte Oberfläche oder eine gefurchte Oberfläche besitzen kann. Bei Beaufschlagung mit Vakuum bewegt sich der Kolben (9, 19, 39, 59, 79, 99) in Richtung Ampullenkopf (3, 33, 53, 73, 93) und schert diesen gegen den Ampullenkörper ab.

Gegebenenfalls kann auch eine gabelförmige Führung (20) an der Innenseite des Kolbens (9, 19, 39, 59, 79, 99) angebracht sein, wobei der Abstand der Stege der Führung (20) gleich groß oder größer als der Durchmesser der Ampulle (2, 32, 52, 72, 92, 132) in der Brechzone ist. Die Führung (20) kann zwischen dem Ampullenkopf (3, 33, 53, 73, 93) und der Ampullenschulter die Ampulle (2, 32, 52, 72, 92, 132) gegen senkrechte Bewegungen entlang der Ampullenachse fixieren. Dadurch kann die Ampulle (2, 32, 52, 72, 92, 132) bei einem Verschieben des Kolbens (9, 19, 39, 59, 79, 99) auf den Ampullenkopf (3, 33, 53, 73, 93) nicht ausweichen. Es wird ein einfacherer Bruch der Ampulle (2, 32, 52, 72, 92, 132) erreicht.

Dabei kann auch vorgesehen sein, dass der Kolben (9, 19, 39, 59, 79, 99) parallel zur Ampullenachse als einseitig geschlossener Hohlzylinder (39, 59, 79) ausgebildet ist, und die Ampulle (2, 32, 52, 72, 92, 132) aufnimmt und zusammen mit der Ampulle (2, 32, 52, 72, 92, 132) oder auch getrennt von der Ampulle (2, 32, 52, 72, 92, 132) in Richtung des Inneren der Kammer (4, 34, 54, 74, 94) verschiebbar ist.

Eine Vorrichtung kann ebenfalls dadurch charakterisiert sein, dass der Kolben (9, 19, 39, 59, 79, 99) auf ein erstes Hebelende eines in der Kammer (4, 34, 54, 74, 94) angeordneten Hebels (40) schiebbar ist, der schräg zur Ampullenachse unterhalb des Ampullenkopfs (3, 33, 53, 73, 93) angeordnet ist, und der mindestens einen Steg (42) senkrecht zur Hebelachse besitzt, wobei der Steg (42) entgegengesetzt zum Ampullenkopf (3, 33, 53, 73, 93) orientiert ist, und der Hebel (40) an dem ersten Hebelende frei in der Kammer (4, 34, 54, 74, 94) liegt und an einem zweiten Hebelende (41) drehbar mit der Kammer (4, 34, 54, 74, 94) verbunden ist.

Wenn durch Beaufschlagung mit Vakuum der Kolben (9, 19, 39, 59, 79, 99) auf das erste Hebelende geschoben wird, dann führt der Hebel (40) eine Drehbewegung aus, wobei der Steg (42) zwangsweise auch eine Drehbewegung und damit eine Scherbewegung gegen den Ampullenkopf (3, 33, 53, 73, 93) ausführt. Dadurch wird der Ampullenkopf (3, 33, 53, 73, 93) vom Ampullenkörper abgeschert.

Eine weitere Ausführungsform ist dadurch charakterisiert, dass der Kolben (79) als einseitig geschlossener Hohlzylinder (79) ausgebildet ist und einen Steg (80) an der geschlossenen Zylinderseite besitzt, dass der in Richtung des offenen Hohlzylinders (79) angeordnete Steg (80) abgeschrägt ist, wobei die Schrägung in Richtung der Längsachse des Kolbens (79) zeigt und der höchste Anstieg der Schrägung innerhalb des Radius des Ampullenquerschnitts ist, und dass die Ampulle (72) im Ampullenbehälter (der Kammer (74)) so angeordnet ist, dass der Ampullenkörper zur Innenwand des Ampullenbehälters (74) einen Abstand hat, der dazu ausreicht, dass der Ampullenkopf (73) abscherbar ist, also zum Beispiel einen Abstand von größer 3 mm hat, wobei der Ampullenkopf (73) in einem nicht elastischen Befestigungshohlzylinder, der einen Innendurchmesser von gleich oder größer dem Ampulenkopf (73) hat, fixiert ist.

Bei Beaufschlagung mit Vakuum bewegt sich der Hohlzylinder (79) in Richtung Ampullenkopf (73). Gleichzeitig bewegt sich der Steg (80) mit in Richtung Ampullenkopf (73). Durch seine Schrägung drückt der Steg (80) dabei den Ampullenkörper senkrecht zur Ampullenachse. Der Ampullenkopf (73) ist in einem Befestigungshohlzylinder gegen Verdrehung fixiert. Dadurch bricht der Ampullenkörper vom Ampullenkopf (73) ab. Durch Krafteinwirkung auf das dem Ampullenkopf (73) entgegengesetzte Ende des Ampullenkörpers genügt infolge der Hebelwirkung nur eine minimale Krafteinwirkung, um den Ampullenkopf (73) von dem Ampullenkörper zu brechen.

Ebenfalls ist ein Prepack-Vakuumzementiersystem gezeigt, bei dem auf ein Fußteil (115) eine Zementpulverkartusche (113, 133) mit mindestens einem Mischorgan (116, 136) und einem Vakuumanschluss (143), einem Ampullenbehälter (1, 31, 51, 91, 111, 131), einem Mittel (112, 140) zur Verbindung der Zementpulverkartusche (113, 133) mit dem Ampullenbehälter (111) und mindestens einer Kammer (4, 34, 54, 74, 94) mit einem Kolben (9, 19, 39, 59, 79, 99) angeordnet ist, wobei der Kolben (9, 19, 39, 59, 79, 99) als Hohlzylinder ausgebildet ist und die Ampulle (2, 32, 52, 72, 92, 132) aufnimmt, wobei der Kolben (9, 19, 39, 59, 79, 99) auf ein erstes Hebelende eines in der Kammer (4, 34, 54, 74, 94) angeordneten Hebels (40) schiebbar ist, der schräg zur Ampullenachse unterhalb des Ampullenkopfs (3, 33, 53, 73, 93) angeordnet ist, und der mindestens einen Steg (42) senkrecht zur Hebelachse besitzt, wobei der Steg (42) entgegengesetzt zum Ampullenkopf (3, 33, 53, 73, 93) orientiert ist, und der Hebel (40) an dem ersten Hebelende frei in der Kammer (4, 34, 54, 74, 94) liegt und an einem zweiten Hebelende (41) drehbar mit der Kammer (4, 34, 54, 74, 94) verbunden ist.

Gezeigt ist auch ein Prepack-Vakuumzementiersystem, das ein Fußteil (115), eine mit Zementpulver gefüllte Zementpulverkartusche (113, 133) mit mindestens einem Mischorgan (116, 136), beispielsweise einem Förderkolben, und einem Vakuumanschluss (143), eine mit einer Monomerflüssigkeit gefüllte Ampulle (2, 32, 52, 72, 92, 132), ein Mittel (112, 140) zur Verbindung der Zementpulverkartusche (113, 133) mit dem Ampullenbehälter (1, 31, 51, 91, 111, 131) und mindestens eine Kammer (4, 34, 54, 74, 94) mit einem Kolben (9, 19, 39, 59, 79, 99) aufweist, wobei der Kolben (9, 19, 39, 59, 79, 99) einen parallel zur Ampullenachse ausgeformten keilförmigen Steg (63) besitzt, wobei eine Schrägung auf der zur Ampullenseite zugewandten Seite angeordnet ist, und sich in der Kammer (4, 34, 54, 74, 94) ein in Längsrichtung zur Ampullenachse ausgebildeter Hebel (60) befindet, dessen Hebelende sich frei beweglich neben dem Ampullenkopf (3, 33, 53, 73, 93) befindet, wobei der Hebel (60) auf der der Ampullenachse abgewandten Seite eine Schrägung besitzt und dass der Hebel (60) mit dem unteren Hebelende (61) beweglich mit der Kammer (4, 34, 54, 74, 94) verbunden ist.

Eine Variante des Prepack-Vakuumzementiersystem ist dadurch charakterisiert, dass auf einem Fußteil (115) eine mit Zementpulver gefüllte Zementpulverkartusche (113, 133) mit mindestens einem Mischorgan (116, 136) und einem Vakuumanschluss (143), eine mit Monomerflüssigkeit gefüllte Ampulle (2, 32, 52, 72, 92, 132), ein Mittel (112, 140) zur Verbindung der Zementpulverkartusche (113, 133) mit dem Ampullenbehälter (1, 31, 51, 91, 111, 131) und mindestens eine Kammer (4, 34, 54, 74, 94) mit einem Kolben (9, 19, 39, 59, 79, 99) angeordnet ist, wobei der Kolben (9, 19, 39, 59, 79, 99) als einseitig geschlossener Hohlzylinder ausgebildet ist und einen Steg (80) an der geschlossenen Zylinderseite besitzt, dass der in Richtung des offenen Hohlzylinders angeordnete Steg (80) abgeschrägt ist, wobei die Schrägung in Richtung der Längsachse des Kolbens (9, 19, 39, 59, 79, 99) zeigt und der höchste Anstieg der Schrägung innerhalb des Radius des Ampullenquerschnitts ist, dass die Ampulle (2, 32, 52, 72, 92, 132) im Ampullenbehälter (1, 31, 51, 91, 111, 131) so angeordnet ist, dass der Ampullenkörper zur Innenwand des Ampullenbehälters (1, 31, 51, 91, 111, 131) einen Abstand von größer 3 mm hat und dass der Ampullenkopf (3, 33, 53, 73, 93) in einem nicht elastischen Hohlzylinder gelagert ist.

Eine Gestaltungsvariante der Vorrichtung ist dadurch charakterisiert, dass der Kolben (99) senkrecht oder annähernd senkrecht gegen den Ampullenkörper schiebbar angeordnet ist, wobei der Ampullenkörper an einen Kolben (99) mit einer Festhaltung (100) fixiert ist. Die Festhaltung (100) kann zum Beispiel gabelförmig ausgeführt sein. Die Festhaltung (100) dient dazu, einerseits eine optimale Kraftübertragung vom Kolben (99) auf den Ampullenkörper zu gewährleisten. Andererseits stabilisiert die Festhaltung (100) die Ampulle (92) während des Transports.

Gemäß einer weiteren Ausführungsform enthält das Prepack-Vakuumzementiersystem ein Fußteil (115), eine mit Zementpulver gefüllte Zementpulverkartusche (113, 133) mit mindestens einem Mischorgan (116, 136) und einem Vakuumanschluss (143), ein Mittel (112, 140) zur Verbindung der Zementpulverkartusche (113, 133) mit dem Ampullenbehälter (1, 31, 51, 91, 111, 131), eine mit Monomerflüssigkeit gefüllte Ampulle (2, 32, 52, 72, 92, 132) und mindestens eine Kammer (4, 34, 54, 74, 94) mit einem Kolben (9, 19, 39, 59, 79, 99), wobei der Kolben (9, 19, 39, 59, 79, 99) senkrecht oder in Wesentlichen senkrecht in oder über dem Ampullenbehälter (1, 31, 51, 91, 111, 131) schiebbar angeordnet ist, wobei der Ampullenkörper an einen Kolben (99) mit einer Halterung (100) fixiert ist.

Eine erfindungsgemäße Vorrichtung umfasst mindestens eine Zementpulverkartusche (113, 133) mit mindestens einem Mischorgan (116, 136), mindestens einen Folienbeutel (132) als Monomerflüssigkeitsbehälter, mindestens eine Kammer (4, 34, 54, 74, 94), mindestens einen Kolben (9, 19, 39, 59, 79, 99)und ist dadurch charakterisiert, dass ein Rohr (140) ausgebildet ist, wobei im Inneren des Rohrs (140) ein Dorn (141) mit Spitze oder ein Dorn (141) mit Schneide angeordnet ist und sich mit seiner Spitze unterhalb des Randes des Rohres (140) befindet und der Folienbeutel (132) mit mindestens einer Außenseite auf dem Rand des Rohrs (140) aufliegt, und dass mindestens ein Vakuumanschluss (143) vorhanden ist, wobei der Vakuumanschluss gleichzeitig als Öffnung für Fluide ausgebildet ist.

Die Vorrichtung basiert darauf, dass durch das Beaufschlagen mit Vakuum der am Rand des Rohres (140) anliegende Folienbeutel (132) in das Rohr (140) gezogen wird. Das Hereinziehen ist auf Grund der Verformbarkeit und Flexibilität des Folienbeutels möglich. Bei dem Hereinziehen in das Rohr (140) wird der Folienbeutel (132) durch den Dorn (141) mit der Spitze oder den Dorn (141) mit der Schneide aufgestochen oder aufgeschnitten, so dass die Monomerflüssigkeit durch das Rohr (140) in Richtung der Zementpulverkartusche (133) fließen kann. Vorteilhaft ist es, wenn der Rand des Rohres (140), an dem der Folienbeutel (132) anliegt, als breiter, flacher Rand ausgeführt wird. Dann ist es möglich, den Folienbeutel (132) direkt auf das Rohr (140) mit einem Klebstoff oder mit einem zweiseitigen ringförmigen Klebeband zu kleben. Damit kann eine dichte Verbindung des Folienbeutels (132) mit dem Rand des Rohrs (140) erreicht werden.

Erfindungsgemäß ist ferner auch ein Prepack-Vakuumzementiersystem, das mindestens eine mit Zementpulver gefüllte Zementpulverkartusche (133) mit mindestens einem Mischorgan (136) und einem Vakuumanschluss (143), mindestens einen mit Monomerflüssigkeit gefüllten Folienbeutel (132) und mindestens einen Kolben (9, 19, 39, 59, 79, 99) enthält, wobei der Kolben (9, 19, 39, 59, 79, 99) als Rohr ausgebildet ist, wobei im Inneren des Rohrs (9, 19, 39, 59, 79, 99) ein Dorn (141) oder ein Dorn (141) mit einer Schneide angeordnet ist und sich mit seiner Spitze unterhalb des Randes des Rohres (9, 19, 39, 59, 79, 99) befindet, dass der Folienbeutel (132) mit mindestens einer Außenseite auf dem Rand des Rohrs (9, 19, 39, 59, 79, 99) aufliegt, und dass mindestens ein Vakuumanschluss vorhanden ist, wobei der Vakuumanschluss gleichzeitig als Öffnung (6, 36, 56, 76, 96) für Fluide ausgebildet ist.

Die in der voranstehenden Beschreibung sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

## Patentansprüche

1. Vakuumzementiersystem umfassend mindestens eine Zementpulverkartusche (133) und ein Behälteröffnungssystem, wobei das Behälteröffnungssystem wenigstens einen Behälter (132) für zumindest eine Flüssigkeit und/oder zumindest ein Pulver, eine statische Öffnungsvorrichtung (141) und eine Auslassöffnung zum Anlegen eines Unterdrucks umfasst, wobei bei Anlegen eines Unterdrucks die Öffnungsvorrichtung (141) relativ zu zumindest einem Teil des Behälters (132) beweglich ist, und das Vakuumzementiersystem ein Mittel (140) zur Verbindung der Zementpulverkartusche (133) mit dem Behälter (132) umfasst, wobei das für die Herstellung eines blasenarmen Zementteigs benötigte Vakuum zusätzlich zum automatischen Öffnen der Behälter (132) genutzt wird, **dadurch gekennzeichnet, dass**
der Behälter (132) eine flexible Wand umfasst und die Öffnungsvorrichtung (141) ein Dorn oder eine Klinge ist, wobei der Behälter (132) beweglich im Behälteröffnungssystem (131) angeordnet ist und/oder die Wand verformbar ist und der Behälter oder ein Teil des Behälters durch Druckdifferenz auf eine statische Öffnungsvorrichtung gedrückt wird und der Differenzdruck direkt und unmittelbar auf den beweglichen Behälter wirkt, so dass sich der Behälter gegen das Behälteröffnungssystem drückt, wobei es sich bei dem Behälter (132) um einen Folienbeutel handelt und eine Wand des Folienbeutels gasdicht an einem die Auslassöffnung bildenden Rohr anliegt und im Inneren des Rohrs (140) ein Dorn (141) mit einer Schneide angeordnet ist, derart, dass bei Anlegen eines Unterdrucks der Folienbeutel in das Innere des Rohrs (140) gesaugt wird und von der Schneide des Dorns (141) aufgeschlitzt wird.

2. Vakuumzementiersystem nach Anspruch 1, **dadurch gekennzeichnet, dass** die Zementpulverkartusche (133) mindestens ein Mischorgan (136), insbesondere einen Förderkolben (136) umfasst.

3. Vakuumzementiersystem nach einem der vorhergehenden Ansprüche, **dadurch gekennzeichnet, dass**
ein statischer Mischer an einer Applikationsöffnung (142) des Vakuumzementiersystems (130) angeordnet ist.

## Claims

1. A vacuum cementation system comprising at least one cement powder cartridge (133) and a container-opening system, wherein the container-opening system comprises at least one container (132) for at least one liquid and/or at least one powder, a static opening device (141) and an outlet opening for applying a negative pressure, wherein, when a negative pressure is being applied, the opening device (141) is mobile with respect to at least a portion of the container (132) and the vacuum cementation system comprises a means (140) for connecting the cement powder cartridge (133) to the container (132), wherein vacuum needed for producing the cement dough with a low bubble content is, in addition, used for automatic opening of the containers (132), **characterised in that**
the container (132) comprises a flexible wall and the opening device (141) is a mandrel or a blade, wherein the container (132) is arranged in the container-opening system (131) such as to be mobile and/or the wall can be deformed and a pressure difference presses the container or a part of the container onto a static opening device and the differential pressure acts directly and immediately on the mobile container such that the container presses against the container-opening system, wherein the container (132) is a film pouch and one wall of the film pouch touches against the tube forming the outlet opening in gas-tight manner and a mandrel (141) having a blade is arranged on the inside of the tube (140) such that, when a negative pressure is being applied, the film pouch is pulled into the inside of the tube (140) and slit open by the blade of the mandrel (141).

2. The vacuum cementation system according to claim 1, **characterised in that** the cement powder cartridge (133) comprises at least one mixing organ (136), in particular a feed plunger (136).

3. The vacuum cementation system according to any one of the preceding claims, **characterised in that**
a static mixer is arranged at an application opening (142) of the vacuum cementation system (130).

## Revendications

1. Système de cimentation sous vide comprenant au moins une cartouche de poudre de ciment (133) et un système d'ouverture de récipient, dans lequel le système d'ouverture de récipient comprend au moins un récipient (132) pour au moins un fluide et/ou au moins une poudre, un dispositif d'ouverture statique (141) et une ouverture de sortie pour l'application d'une dépression, dans lequel le dispositif d'ouverture (141) est mobile par rapport à au moins une partie du récipient (132) en cas d'application d'une dépression, et le système de cimentation sous vide comprend un moyen (140) pour la connexion de la cartouche de poudre de ciment (133) au récipient (132), dans lequel le vide nécessaire pour la fabrication d'une pâte de ciment pauvre en bulles est en outre utilisé pour l'ouverture automatique des récipients (132), **caractérisé en ce que** le récipient (132) comprend une paroi flexible et le dispositif d'ouverture (141) est une broche ou une lame, dans lequel le récipient (132) est disposé de manière mobile dans le système d'ouverture de récipient (131) et/ou la paroi est déformable et le récipient ou une partie du récipient est poussé(e) sur un dispositif d'ouverture statique par différence de pression et la différence de pression agit directement et immédiatement sur le récipient mobile de sorte que le récipient pousse contre le système d'ouverture de récipient, dans lequel il s'agit pour le récipient (132) d'un sachet plastique et une paroi du sachet plastique repose de manière étanche au gaz sur un tuyau formant l'ouverture de sortie et une broche (141) avec un tranchant est disposée à l'intérieur du tuyau (140) de telle sorte que le sachet plastique est aspiré dans l'intérieur du tuyau (140) en cas d'application d'une dépression et est fendu par le tranchant de la broche (141).

2. Système de cimentation sous vide selon la revendication 1, **caractérisé en ce que** la cartouche de poudre de ciment (133) comprend au moins un organe de mélange (136), notamment un piston d'alimentation (136).

3. Système de cimentation sous vide selon une des revendications précédentes, **caractérisé en ce que** un mélangeur statique est disposé sur une ouverture d'application (142) du système de cimentation sous vide (130).
